(19) Europäisches Patentamt
European Patent Office
Office européen des brevets

(11) Publication number: **0 126 043**
**B1**

(12) # EUROPEAN PATENT SPECIFICATION

(45) Date of publication of patent specification: **25.10.89**

(21) Application number: **84850084.9**

(22) Date of filing: **14.03.84**

(51) Int. Cl.[4]: **C 07 H 3/06,** C 07 H 5/06, C 07 H 15/04, C 07 H 15/18, A 61 K 31/70, A 61 K 39/00, G 01 N 33/48

(54) **Carbohydrate derivatives and compositions thereof for therapeutic or diagnostic use.**

(30) Priority: **23.03.83 SE 8301609**

(43) Date of publication of application:
**21.11.84 Bulletin 84/47**

(45) Publication of the grant of the patent:
**25.10.89 Bulletin 89/43**

(84) Designated Contracting States:
**AT BE CH DE FR GB IT LI NL SE**

(56) References cited:
**EP-A-0 035 484**
**EP-A-0 041 897**
**EP-A-0 089 939**
**EP-A-0 089 940**
**WO-A-81/02104**
**WO-A-81/03175**

**CHEMICAL ABSTRACTS, vol. 99, no. 11, 12th September 1983, abstract no. 86273v, Columbus, Ohio, USA D.L. KASPER et al.: "Immunochemical analysis and immunogenicity of the type II group B streptococcal capsular polysaccharide" & J. CLIN. INVEST. 1983, 72(1), 260-269**

(73) Proprietor: **Andersson, Bengt**
**Vänortsgatan 7Al**
**S-431 30 Mölndal (SE)**
(73) Proprietor: **Leffler, Hakon**
**Fagelfängaregatan 11**
**S-414 69 Göteborg (SE)**
(73) Proprietor: **Svanborg-Edén, Catharina**
**Stora Garda 7**
**S-412 70 Göteborg (SE)**

(72) Inventor: **Andersson, Bengt**
**Vänortsgatan 7Al**
**S-431 30 Mölndal (SE)**
Inventor: **Leffler, Hakon**
**Fagelfängaregatan 11**
**S-414 69 Göteborg (SE)**
Inventor: **Magnusson, Göran**
**Dalins väg 1**
**S-233 60 Lund (SE)**
Inventor: **Svanborg, Edén Catharina**
**Stora Garda 7**
**S-412 70 Göteborg (SE)**

(74) Representative: **Inger, Lars Ulf Bosson**
**L + U INGER Patentbyra AB Garvaregatan 12**
**S-262 63 Ängelholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 126 043 B1

Courier Press, Leamington Spa, England.

(56) References cited:

**CARBOHYDRATE RESEARCH, no. 123, 1983, pages 53-61, Elsevier Science Publishers B.V., Amsterdam, NL. S.A. ABBAS et al.: "Synthesis of methyl 3-O and 2-O-(2-acetamido-2-deoxy-beta-D-glucopyranosyl)-alpha-D-galactopyranoside"**

**Description**

The present invention relates to compounds and compositions which are useful for therapeutic treatment of infections as well as prophylaxis and diagnosis in connection therewith.

The technique of the invention is applicable to pathogenic pneumococci. The invention is in particular related to applications directed to *S. pneumoniae*.

A large number of bacterial infections arise by bacterial attack on mucous membranes. In the initial part of the course of inñjection it is essential to the bacterium to be able to bind to epithelium cells. The infectious capacity of bacteria is therefore often directly related to the ability of the bacteria to adhere to epithelium cells.

*S. pneumoniae* is a bacterium which is a frequent cause of ear disorders and sinusitis. The bacterium is present in the flora of healthy nasopharynx and is often cause to inflammation of the middle ear.

Studies have shown that adherence of *S. pneumoniae* to epithelium cells is due to glycoconjugate receptors, and it is an object of the present invention to provide a composition or compound having the ability of replacing the normal receptor function *in vivo* and *in vitro* in relation to pathogenic pneumococci prone to provide disorders in human and animals due to the bacteria.

Another object of the invention is to provide a composition or a compound which can be used diagnostically or which can form the basis for a process for therapeutic or prophylactic treatment of mammals including man.

Another object of the invention is to provide a process for identification and/or quantification of receptor structures of biological materials from mammals including man.

Yet another object of the invention is to provide a process for isolating and purifying bacteria or acceptor structures of bacteria.

The invention is particularly directed to receptor structures for *S. pneumoniae*, but the invention is not limited to this particular bacterium.

By studies and experiments it has been found that the receptor for *S. pneumoniae* contains a structural element having the formula:

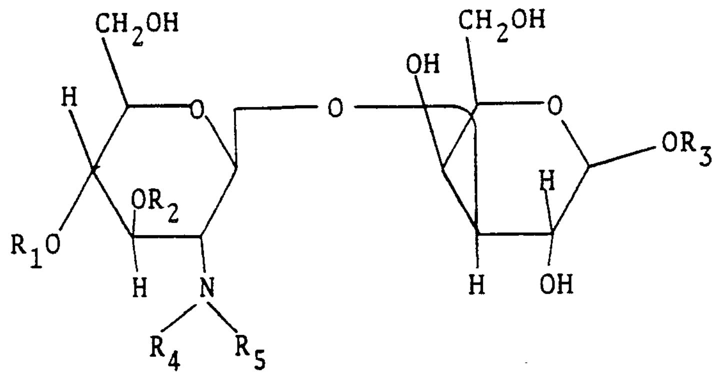

(I)

wherein $R_1$ and $R_2$ are hydrogen or $R_2$ is a galactose residue in β-configuration and $R_3$ is hydrogen, lower alkyl or a carbohydrate residue, whereby $R_3$ may constitute the residue of a natural or synthetic glycoconjugate and when different from hydrogen is in β-configuration, and wherein $R_4$ is hydrogen and $R_5$ is acetyl or $R_4$ and $R_5$ together with the nitrogen atom to which they are attached form a cyclic imide.

In structural formula I $R_2$ and $R_4$ have the meaning of hydrogen in a preferred embodiment. $R_5$ is suitably acetyl.

Preferred meanings of $R_3$ are methyl or a B1→4-Glc residue. If $R_3$ is the residue of a natural or synthetic glycoconjugate, the compound of the invention is a glycoconjugate. The technique concerning the preparation of such glycoconjugates is well developed (see for example references 19 and 20).

In regard to the meaning of $R_4$ and $R_5$ it is preferred that these substituents together with the nitrogen to which they are attached form an imide, for example phthalimide.

The invention thus provides for compounds or compositions which are useful as inhibitors for the adherence of pneumococci to human pharyngeal epithelium cells.

The active substances according to the present invention can be formulated for use in human or veterinary medicine for therapeutic, prophylactic, or diagnostic use. In clinical applications the active constituents are normally administered orally or rectally or by injection in the form of a pharmaceutical preparation containing the active constituents in combination with a pharmaceutically acceptable carrier, which may be solid, semi-solid or liquid, or as a capsule, and such preparations constitute a further aspect of the invention. The compounds may also be used as such without carriers or in the form of an aqueous solution for external use, such as rinsing liquids or for injection. As examples of pharmaceutical preparations there may be mentioned tablets, drops, solutions and suppositories. Usually, the active substance constitutes 0.05 to 99 percent by weight of the preparation, for example from 0.1 to 50 percent for preparations for oral administration.

To prepare pharmaceutical preparations in the form of dosage units for oral application containing a

compound according to the invention the active constituents may be admixed with a solid, pulverulent or other carrier, for example lactose, saccharose, sorbitol, mannitol, starch, such as potato starch, corn starch, amylopectine, a cellulose derivative or gelatine and may also comprise lubricants, such as magnesium or calcium stearate, or polyethylene glycol waxes compressed to form tablets or cores for dragées.

By using several layers of the active drug separated by slowly dissolving layers tablets with delayed release are obtained.

Liquid preparations for oral applications may be in the form of elixir, syrups or suspensions, for example solutions containing from 0.1 to 20 percent by weight of active substance, sugar and a mixture of ethanol, water, glycerol, propylene, glycol and optionally other additives of a conventional nature.

The dose by which the active constituents are administered can vary within broad limits and depends on different factors, such as the seriousness of the disorder, age and weight of the patient and can be individually adjusted. As a conceivable range for the amount of active constituents that may be administered per day there may be mentioned from 0.1 to 2000 mg or from 1 mg to 2000 mg.

In accordance with the invention it has been found that compounds of formula I as defined above have the ability of inhibiting the adherence of bacteria to humane pharyngeal epithelium cells.

Since such adherence in many cases is an essential requisite for the disorders caused by the bacteria these compounds and the corresponding pharmaceutical preparations are useful in therapeutic and/or prophylactic treatment for the purpose of effecting the adherence of the bacteria.

The particular compounds described in the following are with some exceptions all well known as such and can be prepared in accordance with known processes. However, the compounds have not previously been described as possessing properties which would make them medicinally useful.

According to another aspect of the invention there is provided a compound or composition which can be used for identification and/or quantification of the receptor structure in question in biological materials or for purifying bacteria or their acceptor structures. The composition contains one or several structural elements according to the invention above or according to the patent claims covalently or in another manner associated to for example a macromolecular carrier, optionally via a coupling arm. Useful carriers can be synthetic or naturally occurring polypeptides, polysaccharides or other types of polymers or particles. In practice the arm between structural elements and macromolecular carrier can be any of the following:

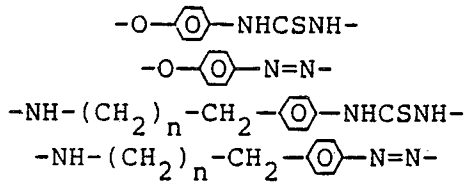

$$-(CH_2)_n-CO-NH-$$

$$-NH-$$

$$-NH(CH_2)_n-CO-NH-$$

$$-O-(CH_2)_nS-R-$$

wherein R is an alkyl or aryl residue (Ref. 21).

According to yet another aspect of the invention there is provided a process for determining the presence of pneumococci in a sample taken from an animal including man. This process is based on the technique of determining the degree of interaction between the bacteria of the sample and a compound or composition in accordance with the present invention. Such interaction may be determined by inhibition or induction of the adherence of the bacteria to cells or other surfaces.

In the above examples n may vary between 1 and 20. The invention will in the following be further described by non-limiting examples.

Bacteria.

Two strains of S. Pneumoniae, EF3114 and EF 10276 (both of the capsular type 6A isolated from the nasopharynx of patients having inflammation in the middle ear) were maintained in a lyophilized state. For adherence test the lyophiles were transferred to blood agar plates and cultivated over night, were then allowed to grow for 18 hours in a synthetic medium (Ref. 1) supplemented with 2 g/l ascorbic acid and such as described in (Ref. 2). The bacteria were harvested by centrifugation and suspended in saline solution (Ref. 3).

Active structures.

The structures of the glycoconjugates and oligosaccharides used are given in table I. Human plasma fibronectine isolated according to known technique (Refs. 4 and 5) was supplied by Dr. A. Simpson, Memphis, Tennessee. E. coli lipopolysacchrides (LPS) from strains of the serotypes 04 and 083 and from mutants having nuclear structures $R_1$, $R_2$, $R_3$ and $R_4$ were prepared by extraction with warm phenol water

(Ref. 6). The R LPS-preparations were supplied by B. and K. Jann, Max-Planck-Institut for Immunobiology, Freiburg, BRD.

The glycolipids neolactotetraosyl ceramide, N-acetylneuraminosyl neolactotetraosyl ceramide and globotetraosylceramide were isolated from humane erythrocytes. For the preparation of neolactotetraosyl ceramide the main ganglioside of human erythrocytes were isolated, N-acetylneuraminosyl neolactotetraosyl ceramide (Ref. 7) was isolated and degraded with Vibrio cholerae neuraminidas (Calbiochem, San Diego, Calif.). The glycolipids were prepared by chloroform methanol extraction, mild alkaline hydrolysis, dialysis, diethylaminoethyl-cellulose chromatogrpahy and silica chromatography (Ref. 8). The glycolipids were further purified as acylated derivatives (Ref. 9) and were found to have a purity of at least 95% according to thin layer chromatography. The known glycolipid structures were confirmed by mass spectrometry, nuclear magnetic resonance (NMR) and degradation methods (Refs. 10, 11, 12 and even 13). For testing the glycolipids were taken from solutions in chloroform-methanol, freed from solvent in a stream of nitrogen, dried over night and suspended in NaCl by means of ultrasound in a water bath.

The tetrasaccharides neolactotetraos and lactotetraos were isolated from human milk (Ref. 14). The octasaccharide was isolated from the urine of a patient having mannosidosis (REf. 15). The known oligosaccharide structures were confirmed by NMR.

Substance *13* below has been prepared synthetically (Ref. 22). Substance *10* is commercially available. Substances *8, 9, 11, 12, 14, 15* and *16* were synthesized in the following manner:

Preparation of substance *8*

Methyl 2-acetamido-2-deoxy-3,4,6-tri-*O*-acetyl-α-*D*-glycopyranoside (Ref. 24) (20)

N-acetyl glycosamine (10 g, 45.2 mmole), Duolite $H^+$ ion exchange mass (20 g), $CaCl_2$ (20 g) and methanol (600 ml) were heated to boiling for 29 h. The admixture was filtered and the residue was washed with methanol. The collected methanol phases were evaporated to dryness and pyridine (250 ml) and acetic anhydride (95 ml) were added to the residue and left for 72 h. Work up gave a yellow syrup which was chromatographed ($SiO_2$, chloroform-methanol 15:1) which gave *20* as a weakly yellow syrup (6.6 g, 40%). N.m.r. data: $^1H(CDCl_3$, TMS) δ 4.77 (d, 1H, $J_{1,2}$ 3.4 Hz, (H—1), 3.43 (s, 3H, MeO), 2.13, 2.06, 2.05, 1.98 (4s, 3H for each, AcO). $^{13}C(CDCl_3$, TMS) δ 98.3 (C—1), 55.4 (MeO).

Methyl 2-acetamido-2-deoxy-4,6-benzyliden-α-*D*-glycopyranoside (*21*).

Substance *20* (6.17 g, 17.1 mmole) was treated with NaOMe (87 mg) in methanol (170 ml) at room temperature for 4 h. T.l.c. (chloroform-methanol 6:1) showed that the reaction was complete. The mixture was treated with Duolit $H^+$ ion exchange mass, filtered and evaporated to dryness which gave *21* (3.16 g, 79%). A small sample was chromatographed ($SiO_2$, chloroform-methanol 8:1) which gave white crystals of *21*. M.p.: 189—191.5°, $[\alpha]_D$ + 137.6° (*c* 0.9, methanol). [Litt. (Ref. 24) M.p. 187—188, $[\alpha]_D$ + 131° (water)]. Dry $ZnCl_2$ (1.79 g, 12.8 mmoles) in benzaldehyde (13.6 g, 128 mmoles) was added and the mixture was stirred under nitrogen gas for 24 h. The reaction mixture was shaken with a mixture of saturated sodium bicarbonate solution (120 ml), ether (120 ml) and petroleum ether (240 ml). Crystalline *21* was formed, which was separated by filtration and washed with water and petroleum ether. The product was dried in vacuum over $P_2O_5$ which gave a residue (4.74 g) which was used directly in the next step. A small sample was recrystallized from methanol: M.p. 259—261°, $[\alpha]_D^{24}$ + 31.2° (*c* 0.8, chloroform). [Litt. (Ref. 25) M.p. 261—262°, $[\alpha]_D^{20}$ + 39.5° (*c* 0.5, chloroform)].

Methyl 2-acetamido-2-deoxy-4,6-benzylidene-3-benzyl-α-*D*-glycopyranoside (*22*).

Barium oxide (18.9 g, 123.5 mmoles) and barium hydroxide octahydrate (7.78 g, 25 mmoles) were added to a suspension of *21* (4 g, 12 mmoles) and benzyl bromide (28.1 g, 164 mmoles) in dry N,N-dimethylformamide (50 ml) at 0°. The reaction mixture was agitated under nitrogen gas, the temperature being allowed to rise to ~22° for 17 h. Chloroform (100 ml) was added and the mixture was filtered through Celite. The filtrate was washed with water, dried ($Na_2SO_4$) and evaporated. The half-crystalline residue was triturated with ether. Filtration and air drying gave crystalline *22* (2.3 g 45%). N.m.r. data: $^1H(CDCl_3$, TMS) δ 5.62 (s, Ø—CH), 4.81(ABq, 2H, $J_{AB}$ 12.0 Hz, Ø—$CH_2$), 4.73 (d, 1H, $J_{1,2}$ 3.8 Hz, H—1), 3.34 (s, 3H, MeO), 1.91 (s, 3H, NAc); $^{13}C(CDCl_3$, TMS) δ 98.8 (C—1).

Methyl 2-acetamido-2-deoxy-3,6-di-*O*-benzyl-α-*D*-glycopyranoside (*23*).

A mixture of *22* (2.28 g, 5.5 mmoles), sodium cyanoborohydride (3.1 g, 49 mmoles), pulverized molecular sieves (2.4 g, 3Å) and freshly distilled tetrahydrofurane (100 ml) under nitrogen gas were supplied dropwise with HCl-saturated ether until the reaction mixture became acid. The mixture was agitated on an ice bath for 45 minutes (t.l.c: chloroform methanol 15:1), poured into ice water and extracted with chloroform (250 + 50 ml). The chloroform solution was washed with sodium bicarbonate solution and water and dried and evaporated. The residue was chromatographed ($SiO_2$, chloroform methanol 15:1) which gave *23* (1.51 g, 36%). M.p. 146—147°, $[\alpha]_D^{24}$ + 88.4° (*c* 1.1, chloroform). N.m.r. data: $^1H(CDCl_3$, TMS) δ 4.67 (d, $J_{1,2}$ 3.6 Hz, H—1), 3.36 (s, 3H, MeO), 1.90 (s, 3H, NAc). $^{13}C(CDCl_3$, TMS) δ 98.8 (C—1), 51.9 (MeO), 23.5 (NAc).

Methyl 2-acetamido-2-deoxy-3,6-di-*O*-benzyl-4-*O*-(2,3,4,6-tetra-*O*-acetyl-β-*D*-galactopyranosyl)-α*D*-glycopyranoside (*24*)

Substance *23* (317 mg, 0.6 mmole) was dissolved in benzene-nitromethane (1:1, 35 ml) $Hg(CH)_2$ (418 mg, 1.65 mmole) then being added. About 10 ml of solvent was evaporated whereafter solution of acetobromogalactose (678 mg, 1.65 mmole recrystallized) in benzene (10 ml) was added dropwise under agitation at 60° (3 h). The reaction mixture was left over night at 40°, cooled, washed (sodium bicarbonate solution, saturated saline solution), dried ($Na_2SO_4$) and evaporated. The residue was chromatographed which gave *24* (281 mg, 63%), which was directly used in the next step.

Methyl 2-acetamido-2-deoxy-4-O-(β-*D*-galactopyranosyl)-α-*D*-glycopyranoside (*8*)

Substance *24* (281 mg, 0.4 mmole) was treated with MeONa (56 mg) in methanol (10 ml) for 2 h (t.l.c.: chloroform methanol 6:1). The reaction mixture was desalted with Duolite $H^+$ ion exchange mass and evaporated. The residue was dissolved in methanol (10 ml) and debenzylated with hydrogen gas (atm. pressure, room temp., Pd/C 10%, 150 mg). The mixture was filtered with Celite, evaporated and chromatographed ($SiO_2$, chloroform-methanol-water 65:35:10, lower phase) which gave *8* (87 mg, 54%). $[\alpha]_D^{21}$ + 80.4° (*c* 0.5, water). N.m.r. data: $^1H$ ($D_2O$) δ 4.79 (d, 1H, J 3.2 Hz, H—1), 4.48 (d, 1H, J 7.1 Hz, H—1'), 3.39 (s, 3H), MeO), 2.03 (s, 3H, NAc); $13_C$ ($D_2O$) δ 105.8 (C—1'), 100.6 (C—1), 58.1 (MeO), 56.1 (C—2), 24.7 (Ac).

Preparation of substance *9*

Methyl 2-acetamido-2-deoxy-3,6-di-*O*-acetyl-4-*O*-(2,3,4,6-tetra-*O*-acetyl-β-*D*-galactopyranosyl)-β-*D*-glycopyranoside (*25*)

Acetylated *D*-lactose oxazoline (Ref. 26, 27) (151 mg, 0.24 mmole) was dissolved in dry methanol (6 ml) containing anhydrous toluene-p-sulfonic acid (10 mg) and stirred at 60° until t.l.c. ($SiO_2$, chloroform-methanol 20:1) showed that the oxazoline had been consumed (15 min.). The mixture was cooled and pyridine (0.5 ml) was added, the solvent being then evaporated with toluene. The residue was chromatographed ($SiO_2$, chloroform-methanol 20:1) which gave *25* (122 mg, 77%). $[\alpha]_D^{25}$ − 18.4° (*c* 1.3, $CDCl_3$). [Litt. (Ref. 28): $[\alpha]_D^{20}$ − 9° (*c* 1.0, $CHCl_3$)]. N.m.r. data: $^1H$($CDCl_3$, TMS) δ 4.50 (d, 1H, $J_{1,2}$ 7.7 Hz, H—1), 4.36 (d, 1H, $J_{1',2'}$7.4 Hz, H—1'), 3.46 (s, 3H, MeO). $^{13}C$($CDCl_3$, TMS) δ 101.8 (C—1'), 101.0 (C—1), (MeO), 23.3 (NAc).

Methyl 2-acetamido-2-deoxy-4-*O*-(β-*D*-galactopyranosyl)-β-*D*-glycopyranoside (*9*).

Substance *25* (77 mg) was deacetylated in the usual manner which gave *9* (43 mg, 92%). $[\alpha]_D^{25}$ − 24.9[9] (*c* 1.5, $D_2O$) [Litt. (Ref. 29): $[\alpha]_D^{22}$ − 23.1° (*c* 0.9, water)]. N.m.r. data: $^1H$ ($D_2O$) δ 4.45 (d, 2H, J. 7.6 Hz, H—1 and H—1'), 3.45 (s, 3H, MeO), 2.02 (s, 3H, NAc). $^{13}C$ ($D_2O$) δ 104.1 (C—1'), 103.1 (C—1), 58.3 (OMe), 23.4 (NAc).

Preparation of substance *11*

Methyl 2,6-di-*O*-benzyl-4-*O*-(2-acetamido-2-deoxy-3,4,6-tri-*O*-acetyl-β-*D*-glycopyranosyl)-β-*D*-galactopyranoside (*30*).

Acetylated *D*-glycose oxazoline (Ref. 31) (1.8 g 6.0 mmoles), methyl 2,6-di-*O*-benzyl-β-D-galactopyranoside (1.5 g, 4.0 mmoles) and toluene-p-sulfonic acid (100 mg) were dissolved in 1,2-dichloroethane (100 ml) and heated at 60° for 6 h (no reaction product was seen by t.l.c. analysis with $SiO_2$, ethyl acetate-isooctane 1:4). Trifluoromethane sulfonic acid (5 drops) was added followed by the oxazoline (2.0 g) and the reaction was continued until all oxazoline had been consumed. Pyridine (2 ml) was added and the mixture was distributed between dichloromethane and water. The organic phase was washed with water, dried ($Na_2SO_4$) and evaporated. The residue was chromatographed which gave *30* (92 mg, 3%). Recrystallization from absolute ethanol gave a material with M.p. 205—206°, $[\alpha]_D$ 0° (*c* 0.5, chloroform). N.m.r. data: $^1H$ ($CDCl_3$, TMS) δ 4.90 (d, 1H, $J_{1',2'}$8.6 Hz, H—1'), 4.26 (d, 1H, $J_{1,2}$ 7.6 Hz, H—1), 3.55 (s, 3H, Meo); $^{13}C$ ($CDCl_3$, TMS) δ 104.5, 101.7 (C—1, C—1'), 56.8 (MeO).

Methyl 2,3,6-tri-*O*-acetyl-4-*O*-(2-acetamido-2-deoxy-3,4,6-tri-*O*-acetyl-β-*D*-glycopyranosyl)-β-*D*-galactopyranoside (*31*)

Substance *30* (92 mg, 0.13 mmole) was hydrogenated (Pd/C), 10%; 4 atm.) in ethanol (100 ml). The mixture was filtered with Celite and evaporated which gave a residue (82 mg) that was acetylated (pyridineacetic anhydride 2:1, 15 ml) at 60° for 2 h. The mixture was evaporated with toluene and the residue was chromatographed ($SiO_2$, chloroform-methanol 50:1) which gave *31* (55 mg, 65%) and having m.p. 189—190°, $[\alpha]_D$ + 4° (*c* 0.2, chloroform). N.m.r. data: $^1H$($CDCl_3$, TMS) δ 5.20 (d, 1H, $J_{1',2'}$8.1 Hz, H—1'), 4.37 (d, 1H, $J_{1,2}$ 7.9 Hz, H—1), 3.51 (s., 3H, MeO); $^{13}C$($CDCl_3$, TMS) δ 101.7, 98.4 (C—1, C—1'), 57.2 (MeO).

Methyl 4-O-(2-acetamido-2-deoxy-β-*D*-glycopyranosyl)-β-*D*-galactopyranoside (*11*).

Substance *31* (42 mg) was deacetylated in the usual manner which gave *11* (18 mg, 77%). $[\alpha]_D^{25}$ − 19° (*c* 0.3, water). N.m.r. data: $^1H$ ($D_2O$, external TMS) δ 4.70 (d, 1H, $J_{1',2'}$ 8.3 Hz, H—1'), 4.30 (d, 1H, $J_{1,2}$ 7.9 Hz, H—1), 3.53 (s, 3H, MeO), 2.04 (s, 3H, NAc); $^{13}C$ ($D_2O$, external TMS) δ 106.5, 104.9 (C—1, C—1'), 59.8 (MeO), 25.1 (NAc).

Preparation of substance *12*

Methyl 2,4,6-tri-*O*-benzyl-3-*O*-2-acetamido-2-deoxy-3,4,6-tri-*O*-acetyl-β-*D*-glycopyranosyl)-β-*D*-galactopyranoside (*26*).

Methyl 2,4,6-tri-O-benzyl-β-D-galactopyranoside (Ref. 30) (1.4 g, 3 mmoles), acetylated D-glycose oxazoline (Ref. 31) (0.99 g, 3 mmoles), tetramethyl urea (0.70 g, 6 mmoles) and trimethylsilyl trifluoromethyl sulfonate (Ref. 32) (0.65 g, 3 mmoles) were dissolved in dry 1,2-dichloroethane (15 ml). The mixture was stirred under nitrogen gas at 80° for 2.5 h, t.l.c. (toluene-ether-methanol 7:7:1) showing that the oxazoline had been consumed. Further oxazoline (0.69 g, 2.1 mmoles) in 1,2-dichloroethane (5 ml) was added and the reaction was continued for 3 h. The mixture was cooled and chloroform (60 ml) added, washed with water (50 ml), saturated sodium-bicarbonate solution (50 ml) and water (50 ml). Each aqueous phase was extracted with chloroform and the collected chloroform extracts were dried ($Na_2SO_4$) and evaporated. The residue was chromatographed ($SiO_2$, toleune-ethylacetate-isopropanol 16:8:1) which gave methyl 2,4,6-tri-O-benzyl-β-D-galactopyranoside (0.35 g, 25% recovered) and the desired product *26* (1.08 g, 45%). N.m.r. data: $^{13}C(CDCl_3$, TMS) δ 104.8 (C—1), 101.8 (C—1′), 56.9 (OMe), 22.8 (NAc).

Methyl 3-*O*-(2-acetamido)-2-deoxy-3,4,6-tri-*O*-acetyl-β-*D*-glycopyranosyl)-β-*D*-galactopyranoside (*27*).

Substance *26* (2.29 g, 2.89 mmoles) was dissolved in ethyl acetate (70 ml) and methanol (20 ml) and hydrogenated (Pd/C, 10%; 4 atm.) for 22 h. The mixture was filtered with Celite, evaporated and chromatographed ($SiO_2$, chloroform-methanol 8:1) which gave *27* (1.24 g, 82%), $[\alpha]_D^{25}$ − 12.3° (*c* 1.1, methanol). N.m.r. data: $^1H(CD_3OD$, TMS) δ 4.86 (d, $J_{1',2'}$8.0 Hz, H—1′), 4.14 (d, $J_{1,2}$ 7.6 Hz, H—1), 3.52 (s. 3H, MeO). $^{13}C(CD_3OD$, TMS) δ 106.0 (C—1), 103.6 (C—1′), 57.3 (MeO), 22.9 (NAc).

Methyl 3-*O*-(2-acetamido-2-deoxy-β-*D*-glycopyranosyl)-β-*D*-galactopyranoside (*12*).

Substance *27* was deacetylated in the usual manner which gave *12*. $[\alpha]_D^{21}$ + 3.6° (*c* 0.4 water). N.m.r. data: $^1H(D_2O)$ δ 4.69 (d, 1H, $J_{1',2'}$8.5 Hz, H—1′), 4.30 (d, 1H, $J_{1,2}$ 8.8 Hz, H—1), 3.56 (s, 3H, MeO), 2.03 (s, 3H, NAc). $^{13}C$ ($D_2O$) δ 106.7 (C—1), 105.5 (C—1′), 60.0 (MeO), 25.0 (NAc).

Preparation of substance *14*

Ethyl 4-O-β-*D*-galactopyranosyl-β-*D*-glycopyranoside (*14*) was prepared from ethanol and lactose octaacetate in the same manner as the corresponding 2-bromoethyl glycoside (Ref. 23). $[\alpha]_D^{21}$ − 1.7° (c 1.0, water); M.m.r. data: $^1H$ (DMSO-$d_6$, 50°, plus $D_2O$) δ 4.28—4.16 (3H, *inter alia* H—1 and H—1′), 1.14 (t, 3H, J7 Hz, $CH_3$). $^{13}C(D_2O$, TSP) δ 105.8, 104.6 (2d, J158 and 155 Hz, C—1 and C—1′), 17.1 ($CH_3$).

Preparation of substance *15*

Methyl 2,4,6-tri-*O*-benzyl-3-*O*-2-acetamido-2-deoxy-3,6-di-*O*-acetyl-4-*O*-(2,3,4,6-tetra-O-acetyl-β-*D*-galactopyranosyl)-β-*D*-glycopyranosyl-β-*D*-galactopyranoside (*28*).

Methyl 2,4,6-tri-O-benzyl-β-*D*-galactopyranoside (Ref. 30) (464 mg, 1 mmole), acetylated D-lactose oxazoline (Ref. 26, 27) (617 mg, 1 mmole), tetramethyl urea (232 mg, 2 mmoles) and trimethyl silyl trifluoromethyl sulfonate (218 mg, 1 mmole) were dissolved in dry 1,2-dichloroethane (10 ml) and treated as for substance *26* which gave *28* (195 mg, 58% based on converted methyl glaactoside). $[\alpha]_D^{23}$ − 14.2° (*c* 1.0, $CDCl_3$). N.m.r. data: $^1H(CDCl_3$, TMS) δ 4.73 (d, 1H, $J_{1',2'}$8 Hz, H—1′), 4.51 (d, 1H, J 8.0 Hz, H—1′′), 4.26 (m, virtual coupling H—1), 3.52 (s, 3H, MeO). $^{13}C(CDCl_3$, TMS) δ 104.8, 101.9, 101.2 (anomeric-C), 56.9 (MeO), 22.8 (NAc).

Methyl 2,4,6-tri-*O*-acetyl-3-*O*-2-acetamido-2-deoxy-3,6-di-*O*-acetyl-4-*O*-(2,3,4,6-tetra-*O*-acetyl-β-*D*-glycopyranosyl-β-*D*-galactopyranoside (*29*).

Substance *28* (180 mg) was dissolved in ethyl acetate (25 ml) and methanol (5 ml) and hydrogenated (Pd/C, 10% 4 atm.) for 24 h. The mixture was filtered with Celite and evaporated to dryness, which gave a product (169 mg) that was then acetylated (acetic anhydride-pyridine 1:2, 3 ml) at room temperature for 16 h. The mixture was evaporated with toluene and the residue was chromatographed ($SiO_2$, chloroform-aceton 5:2) which gave *29* (76%). $[\alpha]_D^{24}$ + 7.2° (*c* 1.3, $CDCl_3$). N.m.r. data: $^1H(CDCl_3$, TMS) δ 4.76, 4.55 4.31 (3d, each 1H, J 7.7, 7.6 and 8.0 Hz, anomeric-H), 3.48 (s, 3H, MeO). $^{13}C(CDCl_3$, TMS) δ 101.8, 101.1, 100.4 (anomeric-C), 56.7 (MeO), 23.2 (NAc).

Methyl 3-*O*-2-acetamido-2-deoxy-4-O-(β-*D*-galactopyranosyl)-β-*D*-glycopyranoxyl-B-*D*-galactopyranoside (*15*).

Substance *29* (109 mg) was deacetylated in the usual manner and the product was chromatographed ($SiO_2$, chloroform-methanol-water 8:6:1), which gave *15* (9 mg, 14%; the major part of the product attached irreversibly on the $SiO_2$-column). $[\alpha]_D^{24}$ + 1.8° (c 0.45, $D_2O$). N.m.r. data: $^1H(D_2O$, 80°) 4.79 (m, virtual coupling, anomeric-H), 4.50 4.30 (2d, each 1H, J7.5 and 7.6 Hz, anomeric-H), 3.56 (s, 3H, MeO), 2.03 (s, 3H, NAc). $^{13}C(D_2O)$ δ 106.7, 105.6, 105.4 (anomeric-C), 60.0 (MeO), 24.8 (NAc).

Preparation of substance *16*

Methyl 2,3,6-tri-O-acetyl-4-O-(2,3,4,6-tetra-O-acetyl-β-D-galactopyranosyl)-β-D-glucopyranoside (30)

A solution of freshly prepared acetobromolactose (72 g, 103 mmol) in a mixture of dry toluene (250 ml)

and methylene chloride (250 ml) was added to a cooled (−55°), stirred solution of methanol (28.5 g, 890 mmol), N,N,N',N'-tetramethylurea (12.9 g, 110 mmol), silver triflate (28.5 g 110 mmol) in dry toluene (250 ml) over a period of 1 h. The reaction mixture was then allowed to attain ambient temperature during 3.5 h, after which time TLC (isooctane: ethyl acetate 1:2) showed the presence of some acetobromolactose. The reaction mixture was cooled again (−15°) and more silver triflate (5.1 g, 20 mmol) and tetramethylurea (3.1 g, 27 mmol) were added. TLC after 1 h showed the reaction to be complete. The mixture was then diluted with methylene chloride (700 ml), filtered through celite. The filtrate was washed with a saturated solution of sodium hydrogencarbonate and water, dried ($Na_2SO_4$), filtered and concentrated to a syrup which was chromatographed ($SiO_2$, isooctane:ethyl acetate 2:1—3:2) to afford pure syrupy *30* (55.8 g, 77.5%) $[\alpha]_D^{25}$ − 11.5° (c 1.3, $CHCl_3$). NMR data: ($CDCl_3$ TMS): $^1H$, δ 5.35 (dd, $J_{4',5'}$1.0 Hz, $J_{3',4'}$3.4 Hz, H—4'), 4.49 (d, $J_{1,2}$ 7.8 Hz, H—1), 4.40 (d, $J_{1',2'}$7.8 Hz, H—1'), 3.49 (s, 3H, $OCH_3$) and 2.16, 2.13, 2.07, 2.054, 2.050, 1.97 (s, 21H, OAc); $^{13}C$, δ 101.3, 101.0 (C—1 and C—1'), 61.9 60.7 (C—6 and C—6'), 57.0 ($OCH_3$).

Methyl β-lactoside (*31*).

A solution of *30* (53.8 g, 82.8 mmol) in methanol (600 ml) containing a catalytic amount of sodium methoxide was stirred at 20° for 3 h. The precipitated product was filtered and washed several times with methanol to give after drying pure *31* (28.2 g, 96%), $[\alpha]_D^{25}$ + 1.7° (c, 1.78, $D_2O$) (Lit[19], $[\alpha]_D^{23}$ + 6.3° (c 3.5, water) for the anhydrous product). $^1H$—NMR ($D_2O$, TSP): δ 4.44 (d, J 7.4 Hz) and 4.40 (d, J 8.4 Hz) (H—1 and H—1'), 3.34 (s, 3H, $OCH_3$). $^{13}C$ ($D_2O$, TSP): 105.9, 105.8 (C—1, C—1'), 63.9, 62.9 (C—6, C—6') and 60.1 ($OCH_3$).

Methyl 4-O-(3,4-isopropylidene-β-D-galactopyranosyl)-β-D-glucopyranoside (*32*).

A suspension of *31* (2.00 g 5.6 mmol) in neat 2,2-dimethoxypropane (45 ml) containing anhydrous p-toluenesulphonic acid (250 mg) was stirred at room temperature for 20 h. The mixture was then diluted with methanol and 5 ml of water was added to obtain a clear solution, which was neutralised with triethylamine (5 ml) and concentrated. The residual semi-solid was chromatographed ($SiO_2$, chloroform: methanol 6:1) to obtain *32* (1.87 g, 84.2%) as an amorphous solid, which crystallised from methanol after several days in the refrigerator; m.p. 222°—223°; $[\alpha]_D^{25}$ + 18.8° (c 1.16, $D_2O$). NMR data: ($D_2O$, TSP): $^1H$, δ4.51 (d, $J_{1',2'}$8.2 Hz, H—1'), 4.42 (d, $J_{1,2}$ 8.0 Hz, H—1), 4.38 (dd, $J_{3',4'}$5.3 Hz, $J_{4',5'}$1.9 Hz, H—4'), 4.22 (dd, $J_{2',3'}$7.5 Hz, H—3'), 3.59 (s, 3H, $OCH_3$), 1.55 and 1.40 )s, 6H, —$C(CH_3)_2$).

Methyl 2,3,6-tri-O-benzyl-4-O-(2,6-di-O-benzyl-3,4-isopropylidene-β-D-galactopyranosyl)-β-D-glucopyranoside (*33*).

Sodium hydride (1.98 g, 50% dispersion in oil) was added in one portion to a stirred solution of 31 (1.36 g, 3.4 mmol) and the suspension was stirred at room temperature for 30 min. after which time benzyl bromide (7.05 g, 41.2 mmol) was added in one portion and the mixture was stirred for 64 h. The excess sodium hydride was then destroyed by dropwise addition of methanol (15 ml), and the reaction mixture was diluted with EtOAc and extracted three times with water, dried ($NaSO_4$), filtered and concentrated. The residual oil was chromatographed ($SiO_2$, isooctane:ethyl acetate 2:1) to obtain *33* (2.50 g, 88.5%) as a clear syrup; $[\alpha]_D^{25}$ + 22.6° (c 1.1, $CHCl_3$). NMR data: ($CDCl_3$, TMS): $^1H$, δ 7.20—7.42 (m, 25H, aromatic), 3.56 (s, 3H, $OCH_3$) 1.40, 1.35 s, 2x3H, $C(CH_3)_2$; $^{13}C$, δ 109.8 $C(CH_3)_2$, 104.8 (C—1'), 101.9 (C—1), 69.0 and 68.3 (C—6, C—6'), 57.2 ($OCH_3$), 28.1 and 26.6 $C(CH_3)_2$.

Methyl 2,3,6-tri-O-benzyl-4-O-(2,6-di-O-benzyl-β-D-galactopyranosyl)-β-D-glucopyranoside (34)

A solution of *33* (15.04 g, 17.8 mmol) in 80% aqueous acetic acid (450 ml) was heated at reflux (100° bath temperature) for 30 min. The solution was then concentrated and co-concentrated with toluene several times to obtain a clear syrup which crystallised upon trituration with diethyl ether: Recrystallisation from diethyl ether and light petroleum (60—80°) gave pure *34* (11.72 g, 81.7%). M.p. 111.5—112.5°, $[\alpha]_D^{24}$ + 22.9° (c 1.26, $CDCl_3$). NMR data: ($CDCl_3$, TMS): $^1H$, δ 7.22—7.42 (m, 25H, aromatic), 3.56 (s, 3H, $OCH_3$), 2.53 (broad d, 1H, OH), 2.44 (broad s, 1H, OH); the latter two protons were $D_2O$-exchangeable. $^{13}C$, δ 104.7 (C—1'), 102.6 (68.7 and 68.3 (C—6 and C—6') and 57.1 ($OCH_3$).

Methyl 2,3,6-tri-O-benzyl-4-O-(2,6-di-O-benzyl-3,6-benzylidene-β-D-galactopyranosyl)-β-D-glucopyranoside (35)

A solution of *34* (11.5 g, 14.3 mmol) and α,α-dimethoxytoluene (10.53 g, 69.3 mmol) in dry tetrahydrofuran (150 ml) containing p-toluenesulphonic acid monohydrate (0.86 g) was stirred at room temperature overnight. The mixture was then neutralised with triethylamine (10 ml) and concentrated. The residual oil was chromatographed ($SiO_2$, isooctane:ethyl acetate 4:1—2.5:1) to give the title compound *35* (11.37 g, 89.1%) as an immobile syrup. NMR data: ($CDCl_3$, TMS): δ 7.21—7.44 (m, 30H, aromatic), 5.96 (s, 1H, benzylidene) and 3.57 (s, 2H, $OCH_3$). This product contained a trace amount of a slightly faster moving substance (TLC), presumably the isomeric product. The product was, therefore, used in the next step without further purification.

Methyl 2,3,6-tri-O-benzyl-4-O-(2,3,6-tri-O-benzyl-β-D-galactopyranosyl)-β-D-glucopyranoside 36 and methyl 2,3,6-tri-O-benzyl-4-O-(2,4,6-tri-O-benzyl-β-D-galactopyranosyl)-β-D-glucopyranoside 37

A solution of the benzylidene acetal *35* (11.37 g, 12.7 mmol) and sodium cyanoborohydride (7.19 g,

114.5 mmol) in dry tetrahydrofuran (170 ml) containing powdered 3Å molecular sieves was cooled to 0°. Hydrogen chloride-saturated diethyl ether was then added until the solution was acidic (pH paper). After 1 h. t.l.c. (isooctane:ethyl acetate 1:2) showed complete reaction. The reaction mixture was then diluted with ethyl acetate and filtered through a celite pad. The filtrate was washed with 10% aqueous hydrogen chloride, water, saturated sodium hydrogencarbonate and then stirred with silica gel for 24 h. filtered and then concentrated. The residue was chromatographed ($SiO_2$, isooctane:ethyl acetate 3:1—2:1) to give syrupy *36* (0.90 g, 79%), identified as the mono-acetyl derivative *38*, $[\alpha]_D^{29}$ + 19.8° (c 1.10, $CHCl_3$). NMR data: ($CDCl_3$, TMS): $^1H$, δ 7.16—7.38 (m, 30H, aromatic), 5.56 (d, $J_{3',4'}$2.2 Hz, H—4'), 3.56 (s, 3H, $OCH_3$) and 2.03 (s, 3H, OAc); and syrupy *37* (6.18 g, 54.3%), $[\alpha]_D^{27}$ + 6.2° (c 1.3, $CHCl_3$). NMR data ($CDCl_3$, TMS): $^{13}C$, δ 104.6 (C—1'), 102.6 (C—1), 80.6 (C—3'), 68.2 and 67.6 (C—6 and C—6'), 57.0 ($OCH_3$).

Methyl 2,3,6-tri-O-benzyl-4-O-(2,4,6-tri-O-benzyl--3-O-[2-acetamido-2-deoxy-3,6-di-O-acetyl-4-O-(2,3,4,6-tetra-O-acetyl-β-D-galactopyranosyl)-β-D-glucopyranosyl]-βD-galactopyranosyl)-β-D-glucopyranoside 38.

A mixture of *37* (4.29 g, 3.84 mmol) lactose-oxazoline (Ref. 26.27) (3.97 g, 6.43 mmol), trimethylsilyl triflate (1.40 g, 6.43 mmol); and N,N,N',N'-tetramethylurea (1.11 g, 9.57 mmol) in dry 1,2-dichloroethane (200 ml) was heated under reflux (bath temperature 70°) under $N_2$. After 3 h more oxazoline (2.00 g, 3.24 mmol), trimethylsilyl triflate (0.62 g, 2.82 mmol) and tetramethylurea (0.50 g, 4.31 mmol) were added and heating was continued for a further 19 h at 60°. The reaction mixture was then cooled, diluted with methylene chloride (300 ml) and washed successively with cold, saturated sodium hydrogencarbonate, cold water and dried ($Na_2SO_4$). Filtration and solvent removal left a brown syrup which was chromatographed ($SiO_2$, toluene:diethyl ether:methanol 14:14:1) to obtain *37* (1.60 g, 37.3%), lactose oxazoline (1.10 g, 18.4%) and a fraction containing *38* together with some impurities. The latter was rechromatographed ($SiO_2$, isooctane:ethyl acetate 1:2) to give essentially pure *38* (2.50 g) as a clear foam. A chromatographically homogenous sample had $[\alpha]_D^{25}$ − 8.9° (c 1.6, $CHCl_3$). NMR data: ($CDCl_3$ TMS): $^1H$ δ 7.22—7.43 (m, 30H, aromatic), 5.35 (d, $J_{3''',4'''}$3.2 Hz, H—4'''), 3.52 (s, 3H, $OCH_3$), 2.15, 2.08, 2.04, 2.02, 1.97, 1.96 and 1.46 (s, 7×3H, O- and N—Ac); $^{13}C$, δ 170.6, 170.4, 170.3, 170.12, 170.07, 169.8, 169.3 (CO), 104.6, 102.5, 102.3, 101.2 (C—1, C—1', C—1'', C—1'''), 62.1, 60.7, (C—6'', C—6'''), 57.0 ($OCH_3$) 53.9 (C—2''), 22.7 ($NCOCH_3$), 20.8, 20.7 and 20.5 ($OCOCH_3$).

Methyl 4-O-{3-O-[2-acetamido-2-deoxy-3,6-di-O-acetyl-4-O-(2,3,4,6-tetra-O-acetyl-β-D-galactopyranosyl)-β-D-gluocopyranosyl]-β-D-galactopyranosyl}-β-D-glucopyranoside 39

A suspension of *38* (2.50 g) and 10% Pd/C (2.50 g) in a mixture of ethyl acetate (50 ml) and methanol (50 ml) was shaken for 24 h under hydrogen (30 psi) in a Parr apparatus. The catalyst was then removed by filtration through a celite pad and the filtrate was concentrated. The residue was chromatographed ($SiO_2$, chloroform:methanol 8:1—6:1) to give *39* (1.20 g 28.8%; from *37*) as a foam. $[\alpha]_D^{25}$ −2.2° (c 1.12, $CDCl_3$) and −4.6° (c 0.83, $CHCl_3$). NMR data: ($CDCl_3$ + $CD_3OD$, TMS): $^1H$, δ 5.36 (dd, $J_{3''',4'''}$3.2 Hz, $J_{4''',5'''}$<1.0 Hz, H—4'''), 4.70, 4.54, 4.35, 4.25 (4d, each 1H, J 8.5, 7.7, 7.9, 7.7 Hz, H1, H1', H1'', H1'''), 3.56 (s, 3H, $OCH_3$), 2.16, 2.12, 2.07, 2.068, 1.98 and 1.94 (s, 7×3H, O— and N—Ac). $^{13}C$, 103.9, 103.7, 101.9, 101.2 (C—1, C—1', C—1'', C—1'''), 62.2, 61.8, 61.4, 60.8 (C—6, C—6', C—6'', C—6'''), 57.2 ($OCH_3$) 54.1 (C—2'') and 22.7 ($NCOCH_3$).

Methyl 4-O-{3-O-[2-acetamido-2-deoxy-4-O-(β-D-galactopyranosyl)-β-D-glucopyranosyl]-β-D-galactopyranosyl}-β-D-glucopyranoside 16

A solution of *39* (650 mg) in methanol (40 ml) containing a catalytic amount of sodium methoxide was left at room temperature for 18 h. T.l.c. (ethyl acetate: acetatic acid: water 2:1:1) examination of the precipitated product indicated incomplete deacetylation. The precipitate was then dissolved in distilled water (15 ml), more sodium methoxide added and the mixture was left in a refrigerator (7°) for 48 h. The mixture was then treated with Duolite C26 ($H^{\pm}$), filtered and concentrated to a glass which was treated with hot aqueous ethanol and the precipitated amorphous product was filtered, redissolved in distilled water, filtered through a millipore membrane (0.5 μm) and freeze-dried to give *16* (328 mg, 68%), $[\alpha]_D^{27}$ +3.8° (c 0.93, $D_2O$). NMR data: ($D_2O$, TSP): δ4.71, 4.49, 4.44, 4.41 (4d, each 1H, J 7.6, 7.6, 7.8, 7.9 Hz respectively H1, H1' H1'', H1'''), 3.58 (s, 3H $OCH_3$) and 2.04 (s, 3H, N—Ac). $^{13}C$, δ 177.7 (CO), 105.9, 105.8, 105.7, 105.6 (C—1, C—1', C—1'', C—1'''), 63.83, 63.78, 62.8, 62.6 (C—6, C—6', C—6'', C—6'''), 60.0 ($OCH_3$), 58.0 (C—2'') and 25.0 (N—Ac).

## TESTS CONCERNING BACTERIAL BINDING

### Adherence to epithelium cells.

Binding to humane nasopharyngeal epithelium cells was investigated *in vitro* as previously described (Ref. 3). Bacterial cells ($10^9$/ml) and pharyngeal epithelium cells from a healthy male donor ($10^4$/ml) were mixed, centrifugated at 1500xg for 10 minutes and incubated at 37°C for 30 minutes. After washing twice in 2.5 ml salines for separating epithelium cells from non-adhering bacteria the number of bacteria adhering at 40 epithelium cells were counted and the average number per cell was determined.

### Haemagglutination

Binding to cells other than epithelium cells can facilitate identification of components taking part in

adherence phenomena. Bacteria binding to erythrocytes result in haemagglutination. The ability of adhering pneumococci to agglutinate erythrocytes was investigated. Newly drained citratized blood was washed and 5% suspensions in saline of humane, bovine, rabbit and guinea pig erythrocytes were prepared. Humane O—, A—, B—, AB— and umbillical cord blood tests were supplied by the blood back, Sahlgrenska Hospital, Gothenburg, Sweden. For enzymatic treatment the suspensions were admixed with Papain (Kebo Grave, Stockholm, Sweden) or neuramidase (SIGMA, St. Louis, MO, USA) till a final concentration of 1 mg/ml and was rotated at 37°C for 30 minutes. After washing twice the suspension was again adjusted to 5%. Bacterial and erythrocyte suspensions (25 µl of each) were admixed on a slide and agglutination was read visually.

Screening in regard to receptor activity of glycoconjugates.

The receptor activity of glycoconjugates or isolated or synethetic oligosaccharides was investigated in two principally different ways.

1. Inhibition of binding by excess of free receptor. For inhibition of adhesion the bacterium concentration giving an average adhesion value of 20—300 bacteria per cell was determined. This inoculate was then preincubated with dilutions of the solutions of potential receptor activity for 15 minutes at 37°C.

Epithelium cells were added and adhesion testing was carried out as described. The adhesion was given in percent of the saline as a control sample.

2. Induction of binding by coating with a receptor. Association of receptor active glycolipids with epithelium cells or erythrocytes can increase binding to epithelium cells already containing the receptor or induce binding of cells which have not previously contained receptor. Rabbit erythrocytes (100 ul of a 5% suspension) were mixed with a neolactotetraosyl ceramide, sialyneolactotetraosylceramide or globotetraosylceramide (500 ul of suspensions of 200 ug/ml) and incubated at 37° for 3 hours with rotation (Ref. 16). After elimination of unbound glycolipid by repeated washing the erythrocyte concentration was again adjusted to 5% and used for haemagglutination. Parallel to the tests regarding inhibition of adherence the humane nasopharyngeal epithelium cells were pretreated with decreasing concentrations of glycolipid, incubated for 2 hours at 37°C, washed and used for testing of adherence.

Results

The neo-lactose series of glycolipids.

The receptor activity of the glycolipids is shown in table II. Pretreatment of the pneumococci with neolactotetraosylceramide inhibited adherence (59% of the saline as a control at 200 µg/ml). Sialyneolactotetraosylceramide showed no effect (table II). Coating of pharyngeal epithelium cells with 200 µg/ml of neolactotetraosylceramide increased adherence (table III), while sialylneolactotetraosylceramide or globotetraosylceramide showed no effect at the same concentration. The structural differences between said compounds is clear from table I.

Oligosaccharides.

From the natural occurring oligosaccharides tested neolactotetraose and lactotetraose were the best inhibitors with a concentration approximately 400 µg/ml for 50% inhibition of adherence of $10^9$ pneumococci (table III). The octasaccharide from humane urine had no effect. N-acetyl-lactose amine and α-methyl-N-acetyl-lactose amine were inactive whereas β-methyl-N-acetyl-lactose amine inhibited adherence at a concentation of about ~5 mg/ml. A number of synthetic oligosaccharides and derivatives were tested for determining receptor specificity. The results are presented in table IV. The results show that th active receptor function is essentially derived from the disaccharide (GlCNAcβ1→3Gal) common to both lacto-N- and lacto-N-neo-tetraos (see substances *5*, *6* and *16* of tables III and IV). GlcNAcβl → 4Galβ-O-Me was inactive (table IV).

In the present disclosure the nomenclature recommended by IUPAC—IUB has been used (Refs. 17 and 18).

TABLE I

Chemical structure of characteristic parts of glycoconjugates and saccharides tested with regard to receptor activity.

| Main compound | Source | Number | Chemical structure of characteristic saccharide part |
|---|---|---|---|
| Glycoconjugate: | | | |
| Humane fibronectine | Human blood | 1 | NeuNAcα2→4(6)Galβ1 → 4GlcNAcβ1 → 2Manα1↘ 6Manβ1→4GlcNAcβ1→ →4GlcNAc-ASN<br>NeuNAcα2→ 4(6)Galβ1 → 4GlcNAcβ1 → 2Manα1↗ 3 |
| Neolactotetraosyl ceramide | -"- | 2 | Galβ1→4GlcNAcβ1→3Galβ1→4Glc-Cer |
| Sialylneolactotetraosyl ceramide | -"- | 3 | NeuNAcα2→3Galβ1→4GlcNAcβ1→3Galβ1→4Glc-Cer |
| Globotetraosylceramide | -"- | 4 | GalNAcβ1→3Galα1→4Galβ1→4Glc-Cer |
| Natural oligosaccharides: | | | |
| Neolactotetraose | Human milk | 5 | Galβ1→4GlcNAcβ1→3Galβ1→4Glc |
| Lactotetraose | -"- | 6 | Galβ1→3GlcNAcβ1→3Galβ1→4Glc |
| "Octasaccharide" | Human urine | 7 | Galβ1→4GlcNAcβ1→2Manα1↘ 6Manβ1→4GlcNAc<br>Galβ1→4GlcNAcβ1→2Manβ1↗ 3 |

TABLE I cont.

| Main compound | Source | Number | Chemical structure of characteristic saccharide part |
|---|---|---|---|
| Synthetic oligosaccharides: | | | |
| α-methyl-N-acetyl-lactose amine | | 8 | Galβ1→4GlcNAcα1-O-Me |
| β-methyl-N-acetyl-lactose amine | | 9 | Galβ1→4GlcNAcβ1-O-Me |
| N-acetyllactose amine | | 10 | Galβ1→4GlcNAc |
| | | 11 | GlcNAcβ1→4Galβ1-O-Me |
| | | 12 | GlcNAcβ1→3Galβ1-O-Me |
| | | 13 | Galβ1→4GlcNAcβ1-O-Et |
| | | 14 | Galβ1→4Glcβ1-O-Et |
| | | 15 | Galβ1→4GlcNAcβ1→3Galβ1-O-Me |
| | | 16 | Galβ1 → 4GlcNAcβ1→3Galβ1→4Glc-β-1-O-Me |
| | | 17 | Galβ1 → 3 GlcNAc-β1-O-Bz |

TABLE II

Effect of glycoconjugates on adherence of pneumococci to humane pharyngeal epithelium cells.

| Compound | Conc. (µg/ml) | Inhibition of adherence[1)] % of control Average | Range | Number of tests | Conc. (µg/ml) | Increase of adherence[2)] % of control Average | Range | Number of tests |
|---|---|---|---|---|---|---|---|---|
| Fibronectine | 100 | 42 | 23-50 | 3 | 1000 | 234 | - | 1 |
| Neolacto-tetraosyl-ceramide | 200 | 59 | 22-100 | 6 | 200 | 190 | 85-244 | 4 |
| Sialylneo-lactotetra-osyl ceramide | 200 | 100 | 93-108 | 3 | 200 | 94 | 65-127 | 3 |
| Globotetra-osyl ceramide | - | | | | 200 | 100 | - | 1 |

1) The bacteria were preincubated with the glycoconjugates before adding epithelium cells.

2) The epithelium cells were preincubated with glycoconjugates, washed and used for adherence testing.

TABLE III

Inhibition of pneumococcal adherence using natural oligo saccharides.

| Oligosaccharide | No. of Table I | Conc. (mg/ml) | Adherence | | |
|---|---|---|---|---|---|
| | | | % of control | | |
| | | | Average | Range | Number of tests |
| Neolactotetraose | 5 | 10 | 1 | 0-2 | 3 |
| | | 5 | 0 | 0-0 | 4 |
| | | 1 | 28 | 14-56 | 5 |
| | | 0.1 | 52 | 36-63 | 4 |
| Lactotetraose | 6 | 5 | 4 | 2-5 | 3 |
| | | 1 | 25 | 14-45 | 3 |
| | | 0.1 | 68 | 55-81 | 2 |
| "Octasaccharide" | 7 | 5 | 71 | 43-116 | 3 |
| | | 1 | 62 | 53-78 | 3 |
| | | 0.1 | 141 | 47-235 | 2 |

TABLE IV

Inhibition of pneumococcal adherence by using synthetic di-, tri- and tetrasaccharides.

| Glycoside | Number of table I | Conc. (mg/ml) | Adherence | | |
|---|---|---|---|---|---|
| | | | % of control Average | Range | Number of tests |
| Galβ1→4GlcNAcα1-O-Me | 8 | 10 | 129 | 111-146 | 2 |
| | | 5 | 98 | 71-127 | 2 |
| | | 1 | 96 | - | 1 |
| Galβ1→4GlcNAcβ1-O-Me | 9 | 10 | 29 | 26-35 | 3 |
| | | 5 | 45 | 32-53 | 4 |
| | | 1 | 96 | 88-103 | 2 |
| Galβ1→4GlcNAc | 10 | 10 | 127 | 12-251 | 4 |
| | | 5 | 144 | 58-335 | 5 |
| | | 1 | 95 | 67-123 | 2 |
| GlcNAcβ1→4Galβ-O-Me | 11 | 10 | 75 | 57-92 | 2 |
| | | 5 | 73 | 65-81 | 2 |
| | | 1 | 72 | 71-72 | 2 |
| | | 0,1 | 65 | 64-66 | 2 |
| GlcNAcβ1→3Galβ-O-Me | 12 | 10 | 4 | 0-11 | 3 |
| | | 5 | 15 | 0-31 | 3 |
| | | 1 | 105 | 80-136 | 3 |
| | | 0,1 | 66 | 65-66 | 2 |

TABLE IV (cont.)

| Glycoside | Number of Table I | Conc. (mg/ml) | Adherence % of control Average | Range | Number of tests |
|---|---|---|---|---|---|
| Galβ1-4GlcNAcβ1-O-Et | 13 | 10 | 31 | 28-34 | 2 |
| | | 5 | 38 | 21-54 | 2 |
| | | 1 | 67 | 59-74 | 2 |
| Galβ1-4Glcβ1-O-Et | 14 | 10 | 63 | 48-77 | 2 |
| | | 5 | 78 | 69-86 | 2 |
| | | 1 | 85 | 83-87 | 2 |
| Galβ1-4GlcNAcβ1-3Gal-β1-O-Me | 15 | 10 | 2 | 0-7 | 3 |
| | | 5 | 11 | 0-25 | 3 |
| | | 1 | 45 | 35-70 | 3 |
| | | 0.1 | 60 | 40-75 | 3 |
| Galβ1 → 4GlcNAcβ1→ 3Galβ1-4 Glc-β-1-O-Me | 16 | 10 | 4 | 1 - 6 | 2 |
| | | 5 | 1 | 0 - 2 | 2 |
| | | 1 | 11 | 10 -12 | 2 |
| | | 0.1 | 104 | 95 -112 | 2 |
| Galβ1 → 3 GlcNAc-β1-O-Bz | 17 | 10 | 8 | 2-12 | 2 |
| | | 5 | 51 | 44-58 | 2 |
| | | 1 | 86 | 82-89 | 2 |
| | | 0.1 | 88 | 87-103 | 2 |

References

1. van de Rijn I., and R. E. Kessler. 1980. Growth characteristics of group A streptococci in a new chemically defined medium. *Infect. Immun.* 27:444.

2. Andersson B., J. Dahmén, T. Frejd, H. Leffler, G. Magnusson, G. Noori, and C. Svanborg Edén. 1983. Identification of an active disaccharide unit of a glycoconjugate receptor for pneumococci attaching to human pharyngeal epithelial cells. *J. Exp. Med.* 158:559.

3. Andersson B., B. Eriksson, E. Falsen, A. Fogh, L. Å. Hanson, O. Nylén, H. Peterson, and C. Svanborg Edén. 1981. Adhesion of *Streptococcus pneumoniae* to human pharyngeal epithelial cells *in vitro*: Differences in adhesive capacity among strains isolated from subjects with otitis media, septicemia, or meningitis or from healthy carriers. *Infect. Immun.* 32: 311.

4. Vuento M. and A. Vaheri. 1979. Purification of fibronectin from human plasma by affinity chromatography under non-denaturing conditions. *Biochem. J. 183:*331.

5. Simpson W. A., D. L. Hasty, J. M. Mason, and E. H. Beachey. 1982. Fibronectin mediates the binding of group A streptococci to human polymorphonuclear leucocytes. *Infect.Immun.* 37:805.

6. Westphal O., and K. Jann. 1965. Bacterial lipopolysaccharides. Extraction with hot phenol-water and further application of the procedure. *In*: Methods in Carbohydrate Chemistry. (Whistler R. C. ed.). Academic Press, New York. 583.

7. Sweeley C. C., and B. Siddiqui. 1977. Chemistry of mammalian glycolipids. *In*: The Glycoconjugates (Horowitz M. I., and W. Pigman, eds.). Academic Press, New York. *I*:459.

8. Karlsson K. —A., B.—E. Samuelsson, and G. O. Steen. 1973. The sphingolipid composition of bovine kidney cortex, medulla and papilla. *Biochem. Biophys. Acta.* 316:317.

9. Handa S. 1963. Blood group active glycolipid from human erythrocytes. *Jpn. J. Exp. Med.* 33:347.

10. Falk K.—E., K.—A. Karlsson, and B.—E. Sammuelsson. 1979. Proton nuclear magnetic resonance analysis of anomeric structure of glycosphingolipids. The globoseries (one to five sugars) *Arch. Biochem. Biophys.* 192:164.

11. Falk K.—E., K.—A. Karlsson, and B.—E. Samuelsson. 1979. Proton nuclear magnetic resonance analysis of anomeric structure of glycosphingolipids. Blood group ABH active substances. *Arch.Biochem.Biophys.* 192:177.

12. Karlsson K.—A. 1978. Mass spectrometric sequence studies of lipid-linked oligosaccharides, blood group fucolipids, gangliosides and related cell surface receptors. *Progr. Chem. Fats Other Lipids.* 16:207.

13. Breimer M. E., G. C. Hansson, K.—A. Karlsson, H. Leffler, W. Pimlott, and B.—E. Samuelsson. 1979. Selected ion monitoring of glycolipid mixtures. Determination of the structure of 8 different blood group type glycolipids in the small intestine of an individual rabbit. *Biomed. Mass Spectrom.* 6:231.

14. Kobata A. 1977. Milk glycoproteins and oligosaccharides. *In*: The Glycoconjugates (Horowitz, M. I., and W. Pigman eds.). Academic Press, London. *I*:423.

15. Lundblad A., S. Sjöblad, and S. Svensson. 1978. Characterization of a penta- and octasaccharide from urine of a patient with juvenile $GM_1$-gangliosiosis. *Arch. Biochem. Biophys.* 188:130.

16. Leffler H., and C. Svanborg Edén. 1980. Chemical identification of a glycosphingolipid receptor for *Escherichia coli* attaching to human urinary tract epithelial cells and agglutinating human erythrocytes. *FEMS Microbiology Letters.* 8:127.

17. IUPAC—IUB combined commission on biochemical nomenclature (CBN). Abbreviations and symbols for chemical names of special interest in biological chemistry. 1967. *Eur.J.Biochem.* *I*:259.

18. IUPAC—IUB commission on Biochemical nomenclature. The nomenclature of lipids. 1978. *Biochem. J.* 171:21.

19. C. P. Stowell and Y. C. Lee, Adv. Carbohydr.Chem.Biochem., 37, 225 (1980).

20. J. D. Aplin and J. C. Wriston, Jr. CRC Critical Rev. Biochem., May 1981, pp. 259—306.

21. J. Dahmén, T. Frejd, G. Magnusson and Ghazi Noori, Carbohydr. Res. 111 (1982) C1—C4.

22. J. Dahmén, T. Frejd, G. Grönberg, T. Lave, G. Magnusson and G. Noori, Carbohydr. Res. in press. (UK 2562).

23. J. Dahmén, T. Frejd, G. Grönberg. T. Lave, G. Magnusson and G. Noori, Carbohydr. Res. in press. (UK 2538).

24. J. Conchie, G. A. Levvy, and C. A. Marsh, Adv. Carbohydr. Chem. *12* (1957) 157.

25. R. Wolfgang and W. Pigman, J. Am. Chem. Soc. 82 (1960).

26. R. Caifu and T. Osawa, Carbohydr. Res. 52 (1976) 179.

27. V. Srivastava, Carbohydr. Res., 103 (1982) 286.

28. T. Takamura, T. Chiba and S. Tejima, Chem. Pharm. Bull. 29 (1981) 2270.

29. R. Kuhn and W. Kirschenlohr, Ann.Chem., 600 (1956) 135.

30. H. Flowers, Carbohydr. Res. 39 (1975) 245.

31. C. Augé and A. Veyrieres, Carbohydr. Res. 46 (1976) 293.

32. T. Ogasa, K. Deppu and S. Nakabayashi, Carbohydr. Res. 93 (1981) C6.

**Claims**

1. A compound for use in the treatment, prophylaxis or diagnosis of bacterial infections caused by pneumococci, characterized in that it has the general formula I:

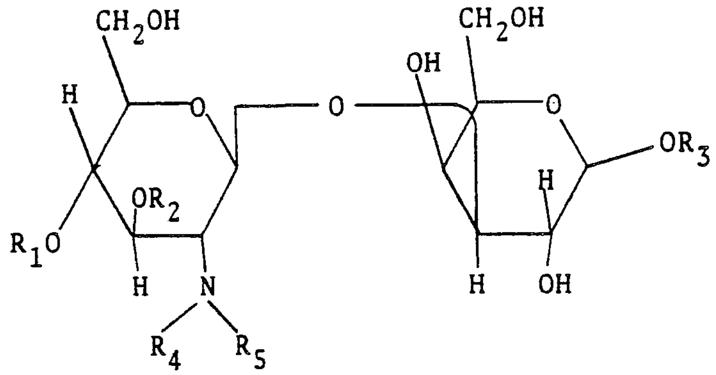

(I)

wherein $R_1$ and $R_2$ are hydrogen or $R_2$ is a galactose residue in β-configuration and $R_3$ is hydrogen, lower alkyl or a carbohydrate residue, whereby $R_3$ may constitute the residue of a natural or synthetic glycoconjugate and when different from hydrogen is in β-configuration, and wherein $R_4$ is hydrogen and $R_5$ is acetyl or $R_4$ and $R_5$ together with the nitrogen atom to which they are attached form a cyclic imide.

2. A compound according to claim 1, characterized in that $R_2$ and $R_4$ are hydrogen.

3. A compound according to claim 1 or 2, characterized in that $R_3$ is lower alkyl.

4. A compound according to claim 1 or 3, characterized in that $R_4$, $R_5$ and said nitrogen atom together form phthalimide.

5. A compound according to claim 1, characterized in that $R_3$ is a β1—4-Glc-residue.

6. A compound according to any preceding claim for use as an inhibitor of adherence of pneumococci to human pharyngeal epithelial cells.

7. A compound according to claims 1 to 4 or 6, characterized in that $R_3$ is methyl.

8. A composition for use in the treatment, prophylaxis or diagnosis of bacterial infections caused by pneumococci, comprising a compound according to any preceding claim in combination with a pharmaceutically acceptable carrier or diluent.

9. A composition according to claim 8, characterized in that it contains a residue of compound I in claim 1 having the formula:

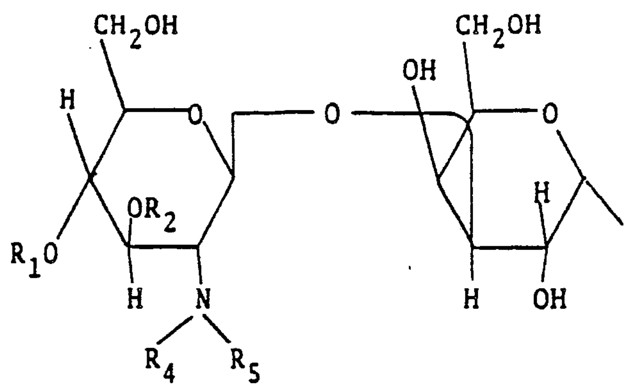

wherein $R_1$, $R_2$, $R_4$ and $R_5$ have the meaning given in claim 1, coupled to a macromolecular carrier.

10. A composition according to claim 9, characterized in that it contains the said residue covalently coupled to a macromolecular carrier via a coupling arm.

11. A composition according to claim 9 or 10, characterized in that as a macromolecular carrier there is used a synthetic or naturally occurring polypeptide, polysaccharide, other polymer or particle.

12. A composition according to any of claim 9—11, characterized in that the coupling arm between structural element and macromolecular carrier is constituted by:

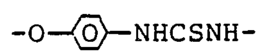

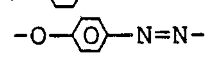

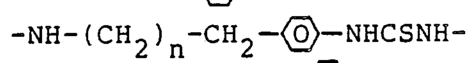

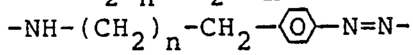

$-(CH_2)_n-CO-NH-$

$-NH-$

$-NH(CH_2)_n-CO-NH-$

$-O-(CH_2)_nS-R-$

wherein R is an alkyl or aryl residue, and wherein n may vary from 1 to 20.

13. The process for indentification or quantification of the compound of any of claims 1—7 in native biological material from mammals including man, characterized by using in the process antibodies, the generation of which has been induced by using the compound of any of claims 1—7 or the composition of any of claims 8—12.

14. A process for purification of acceptor structures of bacteria, characterized by utilising for the purification the affinity between the compound according to any of claims 1—7 or the composition according to any of claims 8—12 and the said acceptor structures of the bacteria.

15. A process for determining the presence of pneumococci in a sample taken from an animal including man, characterized by determining the degree of interaction between the bacteria of the sample and the compound according to any of claims 1—7 or the composition according to any of claims 8—12.

16. A process according to claim 15, characterized by determining the interaction by means of inhibition or induction of the adherence of the bacterium to cells or to other surfaces.

17. The compound Galβ1—4GlcNAcβ1—3Galβ1-O-Me.

18. The compound Galβ1—4GlcNaβ1—3Galβ1—4Glc-β-1-O-Me.

19. The compound Galβ1—3GlcNac-β1-O-Bz.

**Patentansprüche**

1. Verbindung für die Verwendung bei der Behandlung, Prophylaxe oder Diagnose bakterieller Inkektionen, die durch Pneumokokken verursacht sind,. dadurch gekennzeichnet, daß sie die allgemeine Formel I:

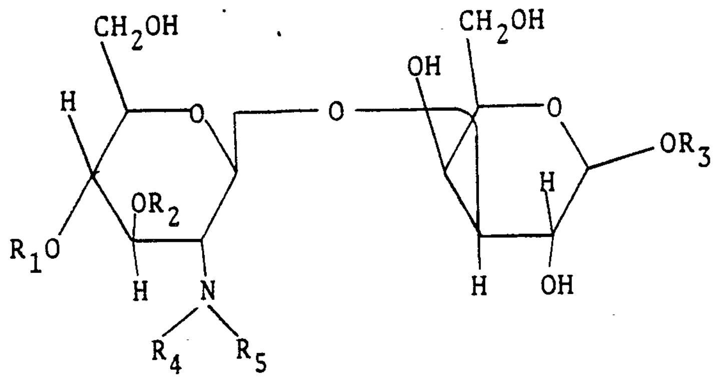

(I)

besitzt, worin $R_1$ und $R_2$ Wasserstoffatome sind oder $R_2$ ein Galactoserest in β-Konfiguration und $R_3$ ein Wasserstoffatom, niedermolekulares Alkyl oder ein Kohlenwasserstoffrest ist, wobei $R_3$ der Rest eines natürlichen oder synthetischen Glucokonjugats sein kann und, wenn es von Wasserstoff verschieden ist, sich in β-Konfiguration befindet und worin $R_4$ Wasserstoff und $R_5$ Acetyl ist oder $R_4$ und $R_5$ zusammen mit dem Stickstoffatom, an das sie gebunden sind, ein zyklisches Imid bilden.

2. Verbindung nach Anspruch 1, dadurch gekennzeichnet, daß $R_2$ und $R_4$ Wasserstoffatome sind.

3. Verbindung nach Anspruch 1 oder 2, dadurch gekennzeichnet, daß $R_3$ niedermolekulares Alkyl ist.

4. Verbindung nach Anspruch 1 oder 3, dadurch gekennzeichnet, daß $R_4$, $R_5$ und das Stickstoffatom zusammen Phthalimid bilden.

5. Verbindung nach Anspruch 1, dadurch gekennzeichnet, daß $R_3$ ein β1—4-Glc-Rest ist.

6. Verbindung nach einem der vorausgehenden Ansprüche für die Verwendung als Inhhibitor für das Anhaften von Pneumokokken an menschlichen Rachenepithelzellen.

7. Verbindung nach den Ansprüchen 1 bis 4 oder 6, dadurch gekennzeichnet, daß $R_3$ Methyl ist.

8. Zusammensetzung für die Verwendung bei der Behandlung, Prophylaxe oder Diagnose bakterieller Infektionen, die durch Pneumokokken verursacht sind, mit einer Verbindung nach einem der vorausgehenden Ansprüche in Verbindung mit einem pharmazeutisch verträglichen Träger oder Verdünnungsmittel.

9. Zusammensetzung nach Anspruch 8, dadurch gekennzeichnet, daß sie einen Rest der Verbindung I in Anspruch 1 mit der Formel:

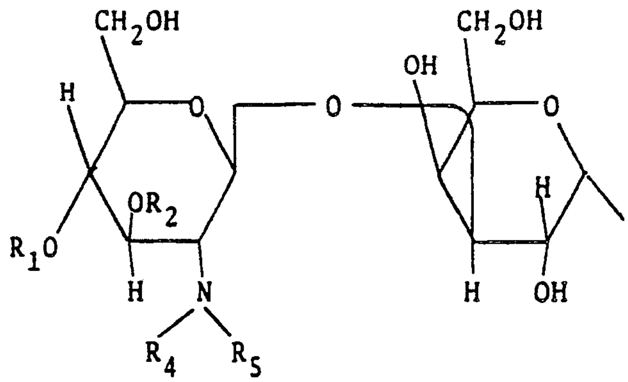

worin $R_1$, $R_2$, $R_4$ und $R_5$ die in Anspruch 1 angegebene Bedeutung haben, an einen makromolekularen Träger gebunden enthält.

10. Zusammensetzung nach Anspruch 9, dadurch gekennzeichnet, daß die den Rest kovalent an einen makromolekularen Träger über einen Bindungsarm gebunden enthält.

11. Zusammensetzung nach Anspruch 9 oder 10, dadurch gekennzeichnet, daß als makromolekularer Träger ein synthetisches oder natürlich vorkommendes Polypeptid, Polysaccharid oder anderes Polymer oder Teilchen verwendet wird.

12. Zusammensetzung nach einem der Ansprüche 9 bis 11, dadurch gekennzeichnet, daß der Bindungsarm zwischen dem Strukturelement und dem makromolekularen Träger von folgenden Gruppen gebildet wird:

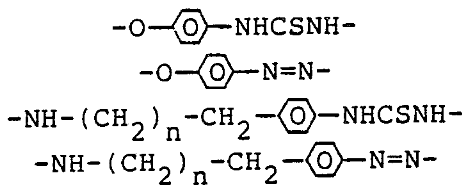

$$-(CH_2)_n-CO-NH-$$

$$-NH-$$

$$-NH(CH_2)_n-CO-NH-$$

$$-O-(CH_2)_nS-R-$$

worin R ein Alkyl- oder Arylrest ist und worin n von 1 bis 20 variieren kann.

13. Verfahren zur Identifizierung oder Quantifizierung der Verbindung nach einem der Ansprüche 1 bis 7 in nativem biologischem Material aus Säugetieren einschließlich Menschen, gekennzeichnet durch die Verwendung von Antikörpern in dem Verfahren, deren Entstehung durch Verwendung der Verbindung nach einem der Ansprüche 1 bis 7 oder der Zusammensetzung nach einem der Ansprüche 8 bis 12 eingeleitet wurde.

14. Verfahren zur Reinigung von Bakterien-Akzeptorstrukturen, gekennzeichnet durch die Ausnutzung der Affinität zwischen der Verbindung nach einem der Ansprüche 1 bis 7 oder der Zusammensetzung nach einem der Ansprüche 8 bis 12 und den Akzeptorstrukturen der Bakterien für die Reinigung.

15. Verfahren zur Bestimmung der Anwesenheit von Pneumokokken in einer von einem Tier einschließlich Menschen abgenommenen Probe, gekennzeichnet durch die Bestimmung des Grades einer Wechselwirkung zwischen den Bakterien der Probe und der Verbindung nach einem der Ansprüche 1 bis 7 oder der Zusammensetzung nach einem der Ansprüche 8 bis 12.

16. Verfahren nach Anspruch 15, gekennzeichnet durch eine Bestimmung der Wechselwirkung mit Hilfe der Hemmung oder des Bewirkens des Anhaftens des Bakteriums an Zellen oder anderen Oberflächen.

17. Die Verbindung Galβ1—4GlcNAcβ1—3Galβ1—O-Me.

18. Die Verbindung Galβ1—4GlcNAcβ1—3Galβ1—4Glc-β-1—O-Me.

19. Die Verbindung Galβ1—3GlcNAc-β1-O-Bz.

## Revendications

1. Composé destiné à s'utiliser dans le traitement, la prophylaxie ou le diagnostic d'infections bactériennes provoquées par des pneumocoques, caractérisé en ce qu'il répond à la formule générale I:

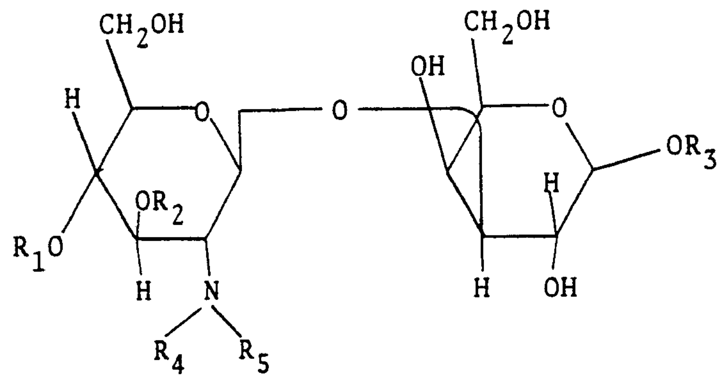 (I)

dans laquelle $R_1$ et $R_2$ représentent de l'hydrogène ou bien $R_2$ est un reste de galactose dans la configuration β, et $R_3$ représente de l'hydrogène, un radical alkyle inférieur ou un reste d'hydrate de carbone, $R_3$ pouvant constituer le reste d'un glyco-conjugué naturel ou synthétique et, lorsqu'il est différent de l'hydrogène $R_3$ se trouve en configuation β, $R_4$ représente de l'hydrogène et $R_5$ le radical acétyle, ou bien $R_4$ et $R_5$ forment, en même temps que l'atome d'azote auquel ils sont attachés, un imide cyclique.

2. Composé suivant la revendication 1, caractérisé en ce que $R_2$ et $R_4$ représentent de l'hydrogène.

3. Composé suivant la revendication 1 ou 2, caractérisé en ce que $R_3$ est un radical alkyle inférieur.

4. Composé suivant la revendication 1 ou 3, caractérisé en ce que $R_4$ et $R_5$ et l'atome d'azote susdit forment ensemble un phtalimide.

5. Composé suivant la revendication 1, caractérisé en ce que $R_3$ représente un rest de βI—4-Glc.

6. Composé suivant l'une quelconque des revendications précédentes, destiné à l'utilisation à titre d'inhibiteur de l'adhérence des pneumocoques sur les cellules épithéliales du pharynx humain.

7. Composé suivant l'une quelconque des revendications 1 à 4 ou 6, caractérisé en ce que $R_3$ est le radical méthyle.

8. Composition destinée à s'utiliser dans le traitement, la prophylaxie et le diagnostic d'infections bactériennes provoquées par des pneumocoques, comprenant un composé suivant l'une quelconque des revendications précédentes en combinaison avec un véhicule ou un diluant acceptable en pharmacie.

9. Composition suivant la revendication 8, caractérisée en ce qu'elle contient un reste de composé I suivant la revendication 1 répondant à la formule:

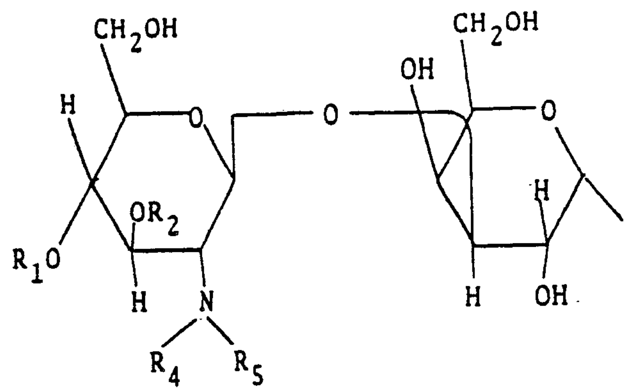

dans laquelle $R_1$, $R_2$, $R_4$ et $R_5$ ont la signification donnée dans la revendication 1, combiné à un véhicule macromoléculaire.

10. Composition suivant la revendication 9, caractérisée en ce qu'elle contient le résidu susdit couplé de manière covalente à un véhicule macromoléculaire par l'intermédiaire d'une branche de combinaison.

11. Composition suivant la revendication 9 ou 10, caractérisée en ce que, comme véhicule macromoléculaire, on utilise un polypeptide, un polysaccharide, d'autres polymères ou particules, naturels ou obtenus par voie de synthèse.

12. Composition suivant l'une quelconque des revendications 9 à 11, caractérisée en ce que la branche de combinaison entre l'élément structural et le véhicule macromoléculaire est constituée par:

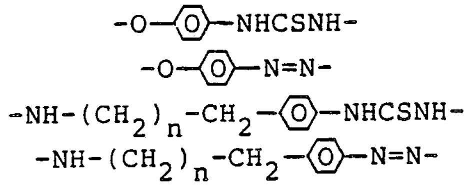

$$-(CH_2)_n-CO-NH-$$

$$-NH-$$

$$-NH(CH_2)_n-CO-NH-$$

$$-O-(CH_2)_nS-R-$$

où R est un reste alkyle ou aryle, et *n* peut varier de 1 à 20.

13. Procédé d'identification ou de quantification du composé de l'une quelconque des revendications 1 à 7 dans une matière biologique native provenant de mammifères, notamment des êtres humains, caractérisé en ce qu'on utilise, dans le procédé, des anticorps dont la génération a été induite par l'utilisation du composé suivant l'une quelconque des revendications 1 à 7 ou de la composition suivant l'une quelconque des revendications 8 à 12.

14. Procédé de purification de structures acceptant les bactéries, caractérisé par l'utilisation, pour la purification, de l'affinité existant entre le composé suivant l'une quelconque des revendications 1 à 7 ou la composition suivant l'une quelconque des revendications 8 et 12, et les structures susdites acceptant les bactéries.

15. Procédé de détermination de la présence de pneumocoques dans un échantillon prélevé sur un mammifère, notamment sur l'homme, caractérisé par la détermination du degré d'interaction entre les bactéries de l'échantillon et le composé suivant l'une quelconque des revendications 1 à 7 ou la composition suivant l'une quelconque des revendications 8 à 12.

16. Procédé suivant la revendication 15, caractérisé par la détermination de l'interaction grâce à l'inhibition ou l'induction de l'adhérence de la bactérie à des cellules ou à d'autres surfaces.

17. Le composé Galβ1—4GlcNAcβ1—3Galβ1-O-Me.

18. Le composé Galβ1—4GlcNAcβ1—3Galβ1—4Glc-β-1-O-Me.

19. Le composé Galβ1—3GlcNac-β1-O-Bz.